(19) **Europäisches Patentamt / European Patent Office / Office européen des brevets**

(11) **EP 2 094 358 B1**

(12) # EUROPEAN PATENT SPECIFICATION

(45) Date of publication and mention
of the grant of the patent:
**07.11.2012 Bulletin 2012/45**

(51) Int Cl.:
*A61P 31/04* [(2006.01)]     *A61K 31/4174* [(2006.01)]
*A61K 31/4178* [(2006.01)]     *A61K 45/06* [(2006.01)]

(21) Application number: **07824591.7**

(22) Date of filing: **15.11.2007**

(86) International application number:
**PCT/GB2007/004370**

(87) International publication number:
**WO 2008/059258 (22.05.2008 Gazette 2008/21)**

(54) **IMIDAZOLES FOR TREATING MULTI-DRUG RESISTANT BACTERIAL INFECTIONS**

IMIDAZOLE ZUR BEHANDLUNG VON MULTI-ARZNEI-RESISTENTEN BAKTERIELLEN INFEKTIONEN

TRAITEMENT D'INFECTIONS BACTÉRIENNES RÉSISTANTES À DE MULTIPLES MÉDICAMENTS

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **16.11.2006 GB 0622839**
**13.06.2007 GB 0711703**
**07.09.2007 US 967843 P**

(43) Date of publication of application:
**02.09.2009 Bulletin 2009/36**

(60) Divisional application:
**12002297.5 / 2 508 231**

(73) Proprietor: **e-Therapeutics plc**
**Long Hanborough**
**Oxfordshire OX29 8LN (GB)**

(72) Inventors:
• **YOUNG, Malcolm Philip**
**Newcastle Upon Tyne NE2 4LZ (GB)**
• **YATES, Catherine Mary**
**Newcastle Upon Tyne NE2 4LZ (GB)**
• **IDOWU, Olusola Clement**
**Newcastle Upon Tyne NE2 4LZ (GB)**
• **CHARLTON, Julie Anne**
**Newcastle Upon Tyne NE2 4LZ (GB)**

(74) Representative: **Gilholm, Stephen Philip**
**IPheions Intellectual Property**
**Buzzard Office**
**The Hawk Creative Business Park**
**Easingwold, York YO61 3FE (GB)**

(56) References cited:
**WO-A-00/10582     WO-A-2005/096990**

• **CARTWRIGHT R Y: "Clotrimazole in the treatment of acute and 'resistant' vaginal candidiasis" POSTGRADUATE MEDICAL JOURNAL, MCMILLAN PRESS, BASINGSTOKE, GB, vol. 50, no. 1 SUP, 1975, pages 90-92, XP008088819 ISSN: 0032-5473**
• **STEVENS D A: "Susceptibility of Candida species to clotrimazole (CZ) in the era of increasing fluconazole (FZ) resistance" ABSTRACTS OF THE GENERAL MEETING OF THE AMERICAN SOCIETY FOR MICROBIOLOGY, AMERICAN SOCIETY FOR MICROBIOLOGY, WASHINGTON, US, vol. 99, 2 June 1999 (1999-06-02), page 321, XP008088821 ISSN: 0067-2777**
• **ANTOLINI M ET AL: "Analogues of 4,5-bis(3,5-Dichlorophenyl)-2-Trifluoromet hyl-1H-Imidaz ole as Potential Antibacterial Agents" BIOORGANIC & MEDICINAL CHEMISTRY LETTERS, OXFORD, GB, vol. 9, no. 7, 5 April 1999 (1999-04-05), pages 1023-1028, XP004162577 ISSN: 0960-894X**
• **MIURA S ET AL: "anti-MRSA activity of 1-(4-chlorophenyl)-3-dichlorophenyl- and 3-trichlorophenyl-2-(1H-imidazol-1-yl)-2-p ropen-1-one derivatives" JOURNAL OF ANTIBIOTICS, JAPAN ANTIBIOTICS RESEARCH ASSOCIATION, TOKYO, JP, vol. 47, no. 10, October 1994 (1994-10), pages 1171-1172, XP008088839 ISSN: 0021-8820**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention provides medicaments and use thereof in methods for the treatment of infections caused or contributed to by specific multi-drug resistant bacterial species.

**BACKGROUND**

Drug resistant microorganisms, especially bacteria, are becoming increasingly problematic as infection rates continue to rise and effective methods of control become more and more limited. Prolific use of antibiotics over the last 50 or so years together with the indiscriminate prescribing of antibiotics and patient non-compliance with treatment regimes, has selected for microorganisms that have developed or acquired ways of overcoming the effects of antibiotics. The transmission and control of drug-resistant organisms is becoming one of the most significant problems within healthcare.

Methicillin-resistant *Staphylococcus aureus* (MRSA) is a specific strain of the *Staphylococcus aureus* bacterium that has developed antibiotic resistance to all penicillins, including methicillin. High levels of resistance to methicillin in Staphylococci, and emerging high levels of resistance to aminoglycosides and ampicillin in Enterococci, have resulted in an increased reliance on vancomycin, particularly as a treatment for MRSA infections. However, this has resulted in the emergence of vancomycin resistant pathogens. Of particular note are strains commonly known as vancomycin intermediately sensitive *Staphylococcus aureus* (VISA), vancomycin resistant *Staphylococcus aureus* (VRSA) and vancomycin resistant Enterococci (VRE). Thus, such vancomycin resistant microorganism strains will not be inhibited by drugs conventionally used in the treatment of MRSA.

Young, elderly and immunocompromised people/patients tend to be at most risk of contracting infections from VISA, VRSA and VRE. Consequently, persistent infections caused or contributed to by drug resistant microorganisms; such as VISA, VRSA and VRE, are often contracted in hospitals (especially intensive care units) and/or nursing homes where the frequent use of antibiotics has created an environment particularly suitable for the survival of drug resistant microorganisms.

VISA, VRSA and VRE are genotypically and phenotypically distinct from vancomycin sensitive gram-positive bacteria, tending to form discrete clonal lineages. The acquisition of mobile genetic elements carrying resistance genes and often virulence determinants results in strains that are often resistant to a number of drugs. Resistance can be specific, i.e. particular to a certain drug or class of drugs or nonspecific in that the resistance applies to a range of drugs, not necessarily related. In the case of VISA, an increase in cell wall thickness is a major contributor to the observed drug resistance.

VISA, VRSA and VRE may be defined as any staphylococcal or enterococcal strain with a vancomycin MIC of 4-8mg/L (VISA) or greater or equal to 8mg/L (VRSA and VRE). These levels of resistance may be due to an increase in cell wall thickness, by the production of cell-wall precursors incapable of binding vancomycin, or via another mechanism. Susceptible gram-positive organisms synthesise cell wall precursors ending in D-ala-D-ala, whereas vancomycin resistant gram-positive organisms synthesise, for example, D-ala-D-lac precursors. The presence of vancomycin resistance in staphylococcal or enterococcal strains may be identified by the measurement of the MIC to vancomycin by broth or agar dilution, or by Etest®, or by the identification of *vanA, vanB, vanC, vanD, vanE, vanG* genes, or similar, by polymerases chain reaction (PCR). The current invention also encompasses the subclass of VISA strains that are heterogeneous VISA (hVISA); these are vancomycin susceptible methicillin-resistant *Staphylococcus aureus* by conventional testing but have a sub population of intermediately resistant cells.

The management of infections caused by VISA, VRSA and VRE reflect these genotypic and phenotypic differences, and requires greater investment in hospital infrastructure, facilities for patient isolation, and infection control measures than for other strains of Staphylococci or Enterococci.

The treatment options for infections contributed to or caused by VISA, VRSA and VRE are now severely limited and there is an urgent need to discover new compounds which inhibit or kill such organisms.

An objective of the present invention is to provide a new and effective treatment for infections contributed to or caused by VISA, VRSA or VRE.

Imidazoles, such as clotrimazole, are a class of compounds that are generally known as antifungal agents. Thus, for example, clotrimazole is indicated in the treatment of vaginal yeast infections, such as candidiasis.

We have now found that clotrimazole has the surprising ability to be highly effective at inhibiting the growth of microorganisms resistant to virtually all currently available antibiotics, such as vancomycin resistant bacteria.

## SUMMARY OF THE INVENTION

In a first aspect, the present invention provides the use of clotrimazole, 1-[(2-chlorophenyl)diphenylmethyl]-1H-imidazole ($C_{22}H_{17}ClN_2$), in the manufacture of an antibacterial agent effective against microorganisms resistant to vancomycin wherein the vancomycin resistant microrganism, is, one or more of VISA, VRSA or VRE.

In particular, the present invention concerns the use of clotrimazole, and/or derivatives thereof, for the preparation of medicaments or for use in methods effective in treating infections contributed to or caused by VISA, VRSA and/or VRE. It should be noted that the Staphylococcal and Enterococcal strains encompassed in the treatment of this invention may also be resistant to other antibiotics not mentioned here.

In addition, the present invention also encompasses uses of various salts and therapeutically active addition salts of the clotrimazole.

It may be convenient or desirable to prepare, purify, and/or handle a corresponding solvate of the compounds described herein, which may be used in any one of the uses/methods described. The term solvate is used herein to refer to a complex of solute, such as a compound or salt of the compound, and a solvent. If the solvent is water, the solvate may be termed a hydrate, for example a mono-hydrate, di-hydrate, tri-hydrate etc, depending on the number of water molecules present per molecule of substrate.

Thus, when a salt of clotrimazole is used the nitrate is particularly preferred. Accordingly, and in one embodiment, the present invention provides a use of clotrimazole and/or clotrimazole nitrate in the manufacture of a medicament for the treatment of an infection contributed to or caused by VISA, VRSA or VRE.

Advantageously, clotrimazole, or derivatives thereof, may be administered orally, topically to the site of an infection, or intravenously. Accordingly, clotrimazole, may be formulated as polymeric nanoparticles such as alginate or polylactide-co-glycolide nanoparticles, or as sterile pharmaceutical compositions comprising a pharmaceutically acceptable carrier or excipient. Such carriers or excipients are well known to one of skill in the art and may include, for example, water, saline, phosphate buffered saline, dextrose, glycerol, ethanol, ion exchangers, alumina, aluminium stearate, lecithin, serum proteins, such as serum albumin, buffer substances such as phosphates, glycine, sorbic acid, potassium sorbate, partial glyceride mixtures of saturated vegetable fatty acids, lactic acid, water salts or electrolytes, such as protamine sulphate, disodium hydrogen phosphate, potassium hydrogen phosphate, sodium chloride, zinc salts, colloidal silica, magnesium trisilicate, polyvinyl pyrrolidone, cyclodextrins, such as α-cyclodextrin, β-cyclodextrin, sulfobutylether$_7$-βcyclodextrin and hydroxypropyl-β-cyclodextrin, cellulose-based substances, polyethylene glycol, sodium carboxymethylcellulose, polyacrylates, waxes, polyethylene-polypropylene-block polymers, polyethylene glycol and wool fat and the like, or combinations thereof.

Clotrimazole, may be administered in combination with another treatment. For example, clotrimazole may be administered in combination with another antibiotic, for example linezolid or quinupristin-dalfopristin, to reduce the likelihood of emergence of antibiotic resistance, or antifungal or antiviral, agents or compounds. Additionally or alternatively, clotrimazole may be administered in combination with a chemotherapeutic agent, an immunostimulatory compound or drug, an oligonucleotide, a cytokine, hormone or the like.

It may be possible to administer clotrimazole or any combined regime as described above, transdermally via, for example, some form of transdermal delivery device. Such devices are advantageous, particularly for the administration of antibiotic compounds, as they may allow a prolonged period of treatment relative to for example, an oral or intravenous medicament.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3249109, US3598122, US4144317, US4262003 and US4307717.

By way of example, clotrimazole may be combined with some form .of matrix .or substrate, such as a non-aqueous polymeric carrier, to render it suitable for use in a transdermal delivery system. The clotrimazole matrix or substrate mixture may be further strengthened by the use of a woven or knit, non-woven, relatively open mesh fabric, to produce a patch, bandage, plaster or the like which may be temporarily attached to a particular region of a patient's body. In this way, while in contact with a patient's skin, the transdermal delivery device releases the compound or substance directly to the site of infection or through the skin as required.

Advantageously, the medicaments and/or methods describe herein may have particular application in institutions housing, sheltering, caring or otherwise holding people or patients vulnerable to or "at risk" of developing or contracting VISA, VRSA or VRE. The medicaments and methods may be particularly useful in hospitals, nursing homes, nurseries and/or schools. More generally, an elderly, young or immunocompromised person or patient may particularly benefit from the medicaments and methods described herein. Moreover, the methods and medicaments of the present invention may be particularly useful to those undergoing a prolonged stay in hospital, for example in an intensive care facility.

Additionally, or alternatively, the medicaments and methods described herein may be useful in community centres, sports facilities, shops, restaurants, cafes or other places where transmission of bacteria, particularly VISA or VRSA, is likely.

In a further embodiment, the methods and medicaments described herein may be used prophylactically as a means to prevent the development of an infection caused or contributed to by VISA, VRSA or VRE. Medicaments and/or methods for prophylactic use may be administered or applied to any person at risk of developing an infection caused or contributed to by VISA, VRSA or VRE. For example, people working in care homes, nursing homes, sports centre, community centres, shops, restaurants, cafes, nurseries and/or schools may require prophylactic treatments.

The compounds provided herein may also be used as sterilising or cleaning aids for use, for example, on surfaces to reduce and/or eliminate contamination by VISA, VRSA or VRE. By way of example, clotrimazole may be prepared for application to any surface suspected of being contaminated by VISA, VRSA or VRE. For example, clotrimazole of the present invention may be added to or diluted in an appropriate excipient or solution prior to use as a sterilising or cleaning agent. Exemplary excipients are described above. Such sterilising or cleaning solutions may be used to decontaminate, for example, furniture, floors, equipment including for example specialised hospital equipment and/or surgical equipment.

In a further embodiment, clotrimazole described herein may be used to eliminate and/or reduce contamination by VISA, VISA or VRE on part of the body, particularly for example, the hands. Clotrimazole may be diluted as an aqueous or non-aqueous solution (dissolved in aqueous, non aqueous or organic solvent) and which may be applied to a body part, for example the hands. Such a solution may find particular application in, for example hospitals, care homes and or nurseries where the prevalence and transmission rates of VISA, VRSA or VRE are often high.

The use of the clotrimazole described herein to treat systemic vancomycin resistant bacterial infections would require the use of a parenteral formulation.

Clotrimazole is well absorbed in humans following oral administration and is eliminated mainly as inactive metabolites. Oral administration of 1.5-3-g doses of clotrimazole gave a half-life of around 3 hours; single or twice daily oral administration may be suitable for the treatment of multiresistant staphylococcal infections with clotrimazole. Less than 1% of the administered dose was detected in urine as active drug after 6 hours.

A clotrimazole topical solution containing, for example, 10 mg/ml (1%) clotrimazole would be suitable for the treatment of multi-resistant staphylococci. Six hours after the application of clotrimazole 1% cream and 1% solution onto intact and acutely inflamed skin, the concentration of Clotrimazole varied from 100 mcg/cm3 in the stratum comeum to 0.5 to 1 mcg/cm3 in the stratum reticulare, and 0.1 mcg/cm3 in the subcutis. Gentle massage of sufficient clotrimazole topical solution into the affected and surrounding skin areas twice a day, in the morning and evening, is suggested for the treatment of multidrug resistant staphylococcal infections.

## DETAILED DESCRIPTION

## METHODS:

In example experiments clotrimazole was dissolved in methanol. Other solvents that may be used include caster oil, pyridine, DMSO and 0.9% saline. For IV administration clotrimazole may be solubilised in polyethoxylated caster oil, or cyclodextrins such as sulfobutylether$_7$-$\beta$cyclodextrin or hydroxypropyl-$\beta$-cyclodextrin and lactic acid. Minimum inhibitory concentrations (MICs) of a range of clinical and control bacterial organisms were measured according to BSAC (British Society for Antimicrobial Chemotherapy) guidelines, described briefly as follows;

## PREPARATION OF AGAR PLATES AND BROTHS

Stock solutions of each agent were prepared using the formula:

$$\frac{1000}{P} \times V \times C = W$$

Where

P = $\mu$g of active compound per mg ($\mu$g/mg)
V = volume required (mL)
C = final concentration of solution (mg/L)
W= weight of agent (mg) to be dissolved in volume V (mL)

Stock solutions were prepared at concentrations of 1000mg/L and 100mg/L. The appropriate amounts of each stock solution were added to separate Petri dishes give the following final concentrations (after the addition of 20mL molten agar): 128, 64, 32, 16, 8, 4, 2, 1, 0.5, 0.25, 0.12, 0.06, 0.03, 0.015mg/L.

Volumes (20mL) of cooled molten IST agar (oxoid) was added to each Petri dish and mixed by swirling.

After drying, the plates were stored at 4°C and protected from light. Plates were used on the day of preparation.

## PREPARATION OF INOCULUM

The test organisms were grown overnight in 5mL IST broth. Using a dilution in 0.9% saline of 1:500 for Gram-negative organisms and 1:100 for Gram-positive organisms, the appropriate agar plates were inoculated using a multipoint inoculator.

## INCUBATION

Plates were incubated at 37°C in air for 18-20 hours.

## INTERPRETATION OF RESULTS

The MIC is the minimum amount of an antibiotic at which there is no visible growth of bacteria. Tiny single colonies or faint hazes were not counted as growth.

## RESULTS:

Clotrimazole demonstrated good activity against strains of Staphylococci and Enterococci with reduced susceptibility to vancomycin (Table 1), but had no significant activity against Gram-negative organisms. Other related imidazoles did not inhibit the growth of any strain tested (Table 2).

## SUMMARY:

Our results demonstrate that clotrimazole is capable of inhibiting the growth of strains of Staphylococci and Enterococci with reduced susceptibility to vancomycin. Other imidazoles such as ketoconazole, fluconazole and bifonazole did not show this activity.

**Table 1.** Minimum inhibitory concentrations (MICs) of miconazole, econazole and clotrimazole against a range of Gram-positive and Gram-negative organisms. Units = μg/mL

| Bacteria | Strain | Clotrimazole | | |
| --- | --- | --- | --- | --- |
| | | replicate 1 | replicate 2 | replicate 3 |
| VISA | VISA 3900 UK | 1.5 | 2.5 | 2.5 |
| VISA | USA/VISA 5827 | 1.5 | 3.5 | 3 |
| *Enterococcus faecium* (VRE) | NCTC 7171 | 4 | 6 | >6 |
| *Enterococcus gallinarum* (VRE) | E1 NCTC 12359 VanC1 | 4 | 6 | >6 |
| *Acinetobacter Baumannii* | ATCC 19606 | >10 | >6 | >6 |
| *Staphylococcus epidermidis* | ATCC 1228 | 3 | 3 | 2.5 |
| *MSA* | USA/VISA 5836 | 1.5 | 3 | 2.5 |
| *Enterococcus faecium* (VRE) | E19 UAA/522 VanB | 1.5 | 3.5 | 3.5 |
| *Enterococcus faecalis* (VRE) | E8 VanA | 6.5 | >6 | >6 |
| *Enterococcus faecium* (VRE) | VanR 8145344C LFE | 3 | 4.5 | 5 |

(continued)

| Bacteria | Strain | Clotrimazole | | |
|---|---|---|---|---|
| | | replicate 1 | replicate 2 | replicate 3 |
| *Enterococcus faecium* (VRE) | E15 VanA ATCC 4147 | 2.5 | 4 | 4.5 |
| *Klebsiella pneumoniae* | ESBL 700603 | >10 | >6 | >6 |
| *Escherichia coli* | NCTC 10418 | >10 | >6 | >6 |
| *Pseudomonas aeruginosa* | NCTC 10662 | >10 | >6 | >6 |

**Table 2.** Minimum inhibitory concentrations (MICs) of other imidazoles against a range of Gram-positive and Gram-negative organisms including clinical MRSA isolates and epidemic strains of MRSA and VISA. Units = $\mu$g/mL

| Bacteria | Strain | Bifonazole | Ketoconazole | Fluconazole |
|---|---|---|---|---|
| VISA | VISA 3900 UK | >128 | 64 | >128 |
| VISA | USA/VISA 5827 | >128 | 64 | >128 |
| *Enterococcus faecium* (VRE) | NCTC 7171 | >128 | 128 | >128 |
| *Staphylococcus epidermidis* | ATCC 1228 | >128 | 64 | >128 |
| VISA | USA/VISA 5838 | >128 | 64 | >128 |
| *Enterococcus faecium* (VRE) | E19 UAA/522 VanB | >128 | 128 | >128 |
| *Enterococcus faecalis* (VRE) | E8 VanA | >128 | >256 | >128 |
| *Enterococcus faecium* (VRE) | VanR B145344C LFE | >128 | 128 | >128 |
| *Enterococcus faecium* (VRE) | E15 VanA ATCC4147 | >128 | 16 | >128 |
| *Escherichia coli* | NCTC 10418 | >128 | >256 | >128 |
| *Pseudomonas aeruginosa* | NCTC 10662 | >128 | >256 | >128 |

## Claims

1. Clotrimazole 1-[(2-chlorophenyl)diphenylmethyl]-1H-imidazole ($C_{22}H_{17}ClN_2$). or a salt thereof for use in the treatment of an infection caused or contributed to by microorganisms resistant to vancomycin wherein the vancomycin resistant microorganism is one or more of VISA, VRSA and VRE.

2. Clotrimazole for use according to claim 1 wherein the clotrimazole salt is selected from the group consisting of clotrimazole and clotrimazole nitrate.

3. Clotrimazole for use according to claim 1 wherein the clotrimazole is suitable for transdermal administration.

4. Clotrimazole for use according to claim 1 wherein the clotrimazole is suitable for administration in combination with an additional therapeutic agent.

5. Clotrimazole for use according to claim 4 wherein the additional therapeutic agent is selected from one or more of an antibiotic agent, an antifungal agent, an antiviral agent, a chemotherapeutic agent, an immunostimulatory agent, an oligonucleotide, a cytokine and a hormone.

6. Clotrimazole for use according to claim 1 wherein the method comprises the prophylactic use of clotrimazole.

7. The use of clotrimazole) or a salt there of as a sterilising or cleaning agent, wherein clotrimazol is used as an antibacterial agent against microorganisms resistant to vancomycin wherein the vancomycin resistant microorganism is one or more of VISA, VRSA and VRE.

**Patentansprüche**

1. Clotrimazol, 1-[(2-Chlorophenyl)Diphenylmethyl)-1H-Imidazol ($C_{22}H_{17}ClN_2$) oder ein Salz davon zur Verwendung in der Behandlung einer Infektion, die von Mikroorgansimen verursacht bzw. zu der diese beigtragen haben, die gegen Vancomycin resistent sind, wobei der gegen Vancomycin resistente Mikroorganismus einer oder mehrere von VISA, VRSA und VRE ist bzw. sind.

2. Clotrimazol zur Verwendung gemäß Anspruch 1, wobei das Clotrimazolsalz ausgewählt ist aus einer Gruppe, die besteht aus Clotrimazol und Clotrimazolnitrat.

3. Clotrimazol zur Verwendung gemäß Anspruch 1, wobei das Clotrimazol für eine transdermale Verabreichung geeignet ist.

4. Clotrimazol zur Verwendung gemäß Anspruch 1, wobei das Clotrimazol für eine Verabreichung in Kombination mit einem weiteren Therapeutikum geeignet ist.

5. Clotrimazol zur Verwendung gemäß Anspruch 4, wobei das weitere Therapeutikum ausgewählt ist aus einem oder mehreren von einem Antibiotikum, einem Antipilzmittel, einem Virostatikum, einem Chemotherapeutikum, einem immunstimulierenden Mittel, einem Oligonukleotid, einem Zytokin und einem Hormon.

6. Clotrimazol zur Verwendung gemäß Anspruch 1, wobei das Verfahren die prophylaktische Verwendung von Clotrimazol umfasst.

7. Verwendung von Clotrimazol oder einem Salz davon als ein Sterilisierungs- oder Reinigungsmittel, wobei das Clotrimazol als antibakterielles Mittel gegen Mikroorganismen, die gegenüber Vancomcyin resistent sind, verwendet wird, wobei der gegen Vancomycin resistente Mikroorganismus einer ist von VISA, VRSA und VRE.

**Revendications**

1. Clotrimazole 1-[(2- Chlorophényl diphényle ;éthyle]-1H -imidazole ($C_{22}H_{17}ClN_2$) ou un sel de celui-ci pour utilisation lors du traitement d'une infection causée ou contribuée par des micro organismes résistant à la vancomycine, **caractérisé en ce que** le micro organisme résistant à la vancomycine est l'un ou plusieurs parmi VISA, VRSA et VRE.

2. Clotrimazole selon la revendication 1, **caractérisé en ce que** le sel de clotrimazole est choisi parmi le groupe consistant en clotrimazole et nitrate de clotrimazole.

3. Clotrimazole selon la revendication 1, **caractérisé en ce qu'**il est adapté à l'administration transdermale.

4. Clotrimazole selon la revendication 1, **caractérisé en ce qu'**il est adapté à l'administration en combinaison avec un agent thérapeutique additionnel.

5. Clotrimazole selon la revendication 4, **caractérisé en ce que** l'agent thérapeutique additionnel est choisi parmi un ou plusieurs de : agent antibiotique, agent antifongique, agent antiviral, agent chimiothérapeutique, agent immunostimulatoire, un oligo nucléotide, une cytokine ou une hormone.

6. Clotrimazole selon la revendication 1, **caractérisé en ce que** le procédé comporte l'utilisation prophylactique de clotrimazole.

7. Utilisation de clotrimazole ou un sel de celui-ci, en tant qu'agent de stérilisation ou de nettoyage, **caractérisée en ce que** le clotrimazole est utilisé en tant qu'agent antibactérien à l'encontre de micro organismes résistant à la vancomycine tandis que le micro organisme résistant à la vancomycine, est l'un parmi VISA, VRSA et VRE.

**EP 2 094 358 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 2185187 A
- US 3249109 A
- US 3598122 A
- US 4144317 A
- US 4262003 A
- US 4307717 A